(19) Europäisches Patentamt
European Patent Office
Office européen des brevets

(11) Veröffentlichungsnummer: **0 151 423**
**A2**

(12) # EUROPÄISCHE PATENTANMELDUNG

(21) Anmeldenummer: 85100539.7

(22) Anmeldetag: 19.01.85

(51) Int. Cl.[4]: C 07 D 209/88
A 61 K 31/40

(30) Priorität: 07.02.84 US 577928

(43) Veröffentlichungstag der Anmeldung: 14.08.85 Patentblatt 85/33

(84) Benannte Vertragsstaaten: AT CH LI LU NL SE

(71) Anmelder: F. HOFFMANN-LA ROCHE & CO. Aktiengesellschaft

CH-4002 Basel(CH)

(72) Erfinder: Berger, Leo
7 Parkway
Montclair, N.J.(US)

(72) Erfinder: Coffen, David Llewellyn
270 Ridgewood Avenue
Glen Ridge, N.J.(US)

(72) Erfinder: Manchand, Percy
15 Prescott Avenue
Montclair, N.J.(US)

(72) Erfinder: Mandeville, W. Harry
440 Lafayette Avenue
Wyckoff, N.J.(US)

(74) Vertreter: Mahé, Jean et al,
Postfach 3255 Grenzacherstrasse 124
CH-4002 Basel(CH)

(54) **Verfahren zur Herstellung eines Carbazolderivates.**

(57) Neue Verbindungen der Formel

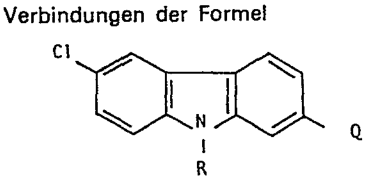

V

worin R Wasserstoff, Acetyl oder Propionyl und Q Acetyl ist oder R Wasserstoff und Q $C(H,CH_3)OC(O)R^2$ oder $C(CN,CH_3)OSi(R^3,R^4,R^5)$ ist, worin $R^2$ Wasserstoff, $C_{1-10}$-Alkyl, $C_{2-10}$-Alkenyl, $C_{4-7}$-Cycloalkyl, Aryl oder eine 5- oder 6-gliedrige heterocyclische Gruppe ist, und $R^3$, $R^4$ und $R^5$ geradkettige $C_{1-4}$-Alkylgruppen sind, ihre Herstellung aus 2-Acetyl-carbazolen und ihre Verwendung zur Herstellung der 6-Chlor-α-methylcarbazol-2-essigsäure.

EP 0 151 423 A2

Croydon Printing Company Ltd

F.Hoffmann-La Roche & Co. Aktiengesellschaft, Basel/Schweiz



RAN 4001/108

## Verfahren zur Herstellung eines Carbazolderivates

Die Erfindung betrifft ein neues Verfahren zur Herstellung der 6-Chlor-α-methylcarbazol-2-essigsäure, ein bekanntes antiinflammatorisches Mittel, sowie neue Zwischenprodukte in dieser Herstellung.

Dieses Verfahren ist dadurch gekennzeichnet, dass man

a) eine Verbindung der Formel

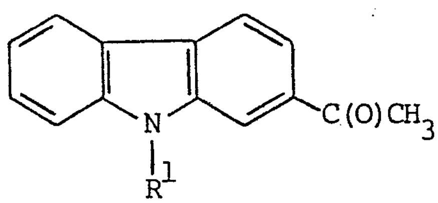

I

worin $R^1$ Wasserstoff, Acetyl oder Propionyl ist, chloriert und entweder

Mé/15.1.85

b) die entstandene Verbindung der Formel

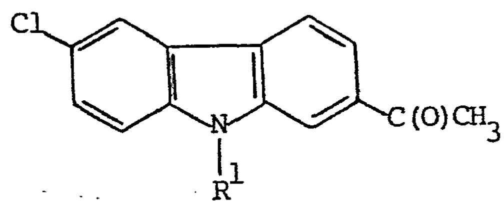

II

worin $R^1$ die obige Bedeutung hat

mit einem Reduktionsmittel umsetzt

c) die entstandene Verbindung, 2-(1-Hydroxyäthyl)-6-chlorcarbazol, acyliert und

d) die entstandene Verbindung der Formel

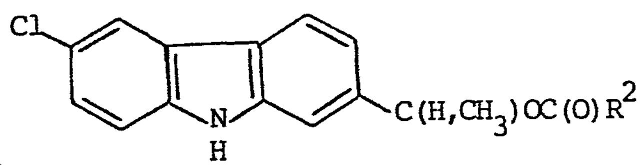

III

worin $R^2$ Wasserstoff, $C_{1-10}$-Alkyl, $C_{2-10}$-Alkenyl, $C_{4-7}$-Cycloalkyl, Aryl oder ein 5- oder 6-gliedrige heterocyclische Gruppe ist,

mit einem Verschiebungsmittel umsetzt, oder

e) die Verbindung der Formel II, worin $R^1$ Wasserstoff ist, 2-Acetyl-6-chlorcarbazol, mit Tosylmethylisocyanid in Gegenwart von Kalium-t-butoxid umsetzt oder

f) 2-Acetyl-6-chlorcarbazol mit einem Tri-(geradkettigem $C_{1-4}$-alkyl)-silylcyanid umsetzt und

g) die entstandene Verbindung der Formel

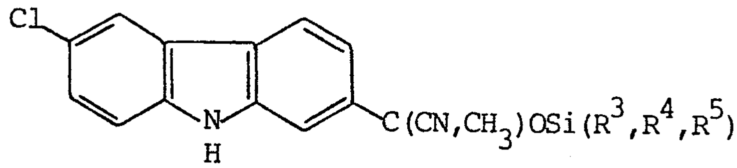

IV

worin $R^3$, $R^4$ und $R^5$ geradkettige $C_{1-4}$-Alkylgruppen sind,
mit Zinnchlorid umsetzt und

h) das Produkt der Verfahrensstufe d), e) oder g), 2-(1-Cyanoäthyl)-6-chlorcarbazol, hydrolysiert.

Im Rahmen der Erfindung bedeuten die Ausdrücke "Alkyl" und "Alkenyl" geradkettige oder verzweigte Gruppen wie Isopropyl, Butyl, t-Butyl, Neopentyl, Pentyl oder Heptyl, und Aethenyl, Propenyl, Butenyl, Pentenyl, Hexenyl, Isopropenyl oder Isobutenyl. "Cycloalkyl" bezeichnet Cyclobutyl, Cyclopentyl, Cyclohexyl und Cycloheptyl. "Aryl" bezeichnet eine aromatische Gruppe ohne Heteroatome im Ring selbst, z.B. Phenyl oder Naphthyl, die gegebenenfalls durch Nitro, Fluor, Chlor, Brom, Jod, Alkoxy oder Alkyl substituiert ist, z.B. 4-Aethylphenyl, 4-Nitrophenyl, 4-Chlorphenyl, 4-Methoxyphenyl oder 1-(3-Methylnaphthyl). Eine "5- oder 6-gliedrige heterocyclische Gruppe" bezeichnet eine aromatische oder nicht aromatische Gruppe mit bis zu drei, vorzugsweise einem oder zwei Heteroatome, z.B. Pyridyl, Pyrimidinyl, Imidazolyl, Furyl, Thiazolyl, Oxazolyl, Isoxazolyl, Pyrazinyl, Piperidyl, Tetrahydrofuranyl, Chinolinyl, Isochinolinyl, Chinazolinyl, 1,2,4-Triazinyl, Benzimidazolyl oder Pyridazinyl, gegebenenfalls substituiert wie oben für Arylgruppen beschrieben, z.B. 2-Chlor-, 2-Nitro- oder 2-Methoxypyridyl oder 2-Methyltetrahydrofuranyl. "Acyloxy" bezeichnet eine Alkanoyloxy- oder Aroyloxygruppe oder den Rest einer heterocyclischen Carbonsäure, z.B. Formyloxy, Acetoxy, Propionyloxy, Butyryloxy, Valeryloxy, Hexanoyloxy, Octanoyloxy,

Decanoyloxy, Benzoyloxy, 4-Methylbenzoyloxy oder 2-Thienoyloxy.

Die Verbindungen der Formel I sind bekannt oder können in Analogie zu bekannten Verbindungen hergestellt werden. Zum Beispiel wird Carbazol mit Propionsäureanhydrid oder Propionsäurechlorid in Gegenwart einer Base, wie Pyridin oder Lutidin, unter Rückfluss umgesetzt. Das entstandene 9-Propionylcarbazol wird mit Acetylchlorid in Gegenwart eines Friedel-Crafts-Reagenz, z.B. wasserfreiem $AlCl_3$, zu 9-Propionyl-2-acetylcarbazol umgesetzt.

In einer bevorzugten Durchführungsform der Chlorierungsstufe a) wird 2-Acetyl-9-propionylcarbazol oder 2,9-Diacetylcarbazol mit Sulfurylchlorid, Natriumhypochlorit oder Trichlorisocyansäure in einem polaren Lösungsmittel, wie Essigsäure, Triäthylphosphat, 1,2-Dichloräthan oder Dimethylformamid (DMF) umgesetzt. In einer besonders bevorzugten Ausführungsform wird diese Reaktion mit Trichlorisocyansäure in DMF bei etwa 40 bis 60°C, zweckmässig etwa 50°C, innerhalb etwa 2-10 Stunden durchgeführt. Unter diesen Umständen wird nur wenig oder kein 3,6-Dichlornebenprodukt gebildet.

In einer weiteren bevorzugten Durchführungsform der Verfahrensstufe a) wird 2-Acetylcarbazol mit 1-Chlorbenzotriazol in einem polaren Lösungsmittel, wie Methylenchlorid oder 1,2-Dichloräthan, bei etwa Raumtemperatur innerhalb etwa 1-18 Stunden zu 2-Acetyl-6-chlorcarbazol chloriert. In einer besonders bevorzugten Ausführungsform wird 2-Acetylcarbazol mit Trichlorisocyansäure in Triäthylphosphat in Gegenwart einer Lewis-Säure, wie Aluminiumchlorid oder zweckmässig Bortrifluoridätherat oder Fluororsäure, bei etwa -10 bis +25°C, etwa 1-7 Stunden behandelt. 2-Acetyl-6-chlorcarbazol wird durch Abdampfen des Lösungsmittels, Behandlung des Reaktionsgemisches mit Natriumhydroxid zur Entfernung der Cyanursäure und Kristallisation des Produktes aus der

Gemisch von DMF und Acetonitril isoliert.

Alternativ kann 2-Acetyl-6-chlorcarbazol durch Hydrolyse des 6-Chlor-2,9-diacetylcarbazols, z.B. mit einer Base wie Natrium- oder Kaliumhydroxid in einem Alkohol, wie Aethanol oder Propanol, bei Rückflusstemperatur hergestellt werden.

Die Reduktionsstufe b) wird vorzugsweise mit Natriumborhydrid allein oder mit konzentriertem Natriumhydroxid durchgeführt. Weitere Reduktionsmittel sind Lithiumaluminiumhydrid, Lithiumborhydrid oder Diisobutylaluminiumhydrid. Die Reduktion kann in einem Lösungsmittel, wie einem niederen Alkanol, z.B. Methanol, Aethanol oder Propanol durchgeführt werden. Die Temperatur des Reaktionsgemisches ist nicht kritisch. Vorzugsweise wird die Reduktion mit 10%-igem Natriumborhydrid, basiert auf dem Gewicht der Verbindung der Formel II, in Aethanol unter Rückfluss durchgeführt. Das entstandene 2-(1-Hydroxyäthyl)-6-chlorcarbazol wird durch Abdampfen des Lösungsmittels, Zerreiben des resultierenden Produktes mit Wasser und Trocknen isoliert.

Zur Durchführung der Acylierungsstufe c) kann man ein von der Säure $R^2C(O)OH$, worin $R^2$ die obige Bedeutung hat, abgeleitetes Säurechlorid oder Säureanhydrid, wie Acetanhydrid, Benzoylchlorid oder das gemischte Anhydrid von Ameisensäure und Essigsäure, verwenden. Die Acylierung wird zweckmässig in Gegenwart eines nicht-hydroxylischen Lösungsmittels, wie Aethylacetat, Tetrahydrofuran, Aether, Dioxan, Toluol oder Methylenchlorid, durchgeführt. Den Acylierungskatalysator kann man z.B. in einem Verhältnis von etwa 0,05 bis 5 Gewichtsprozente basiert auf dem Gewicht der Reaktanten verwenden. Bei Verwendung eines Säurechlorids ist ein Acylierungskatalysator nicht nötig. Zweckmässig werden tertiäre Amine, wie 4-Dimethylaminopyridin, als Katalysator verwendet. Die Temperatur der Acylierung ist nicht kritisch. Die entstandenen Verbindungen der Formel III können in an sich bekannter Weise, z.B. durch Abdampfen des Lösungsmittels und Zerreiben des Rückstandes mit Wasser isoliert werden.

Bevorzugte Verbindungen der Formel III sind diejenigen, worin $R^2$ Alkyl, Phenyl oder substituiertes Phenyl sind. Beispiele von Verbindungen der Formel III sind 2-(1-Acetoxyäthyl)-, 2-(1-Benzoyloxyäthyl)- und 2-(1-Formyloxyäthyl)-6-chlorcarbazol.

Die Reaktionsstufe d) kann man mit einem Verlagerungsmittel, wie einem Alkalimetallcyanid, z.B. Kalium-, Lithium- oder Natriumcyanid, in einem polaren aprotischen Lösungsmittel wie Dimethylsulfoxid (DMSO), DMF, N-methyl--pyrrolidon, Sulfolan oder Hexamethylphosphoramid, bei einer Temperatur im Bereich von etwa 100 bis 200°C, vorzugsweise etwa 125 bis 155°C, jedoch unterhalb der Zersetzungstemperatur des Lösungsmittels durchführen. Die bevorzugte Reaktionstemperatur in DMSO ist 125 bis 135°C und in DMF die Rückflusstemperatur. DMSO ist bevorzugt wenn man Natriumcyanid verwendet und DMF wenn man Lithiumcyanid verwendet. Die Reaktion wird zur Vermeidung der Bildung des entsprechenden Alkohols, nämlich 2-(1-Hydroxyäthyl)-6-chlorcarbazol, unter praktisch wasserfreien Bedingungen durchgeführt. Zweckmässig wird vor der Reaktion das Lösungsmittel und das Alkalimetallcyanid getrocknet und die Reaktion wird unter einer inerten Atmosphäre, z.B. Stickstoff oder Argon, durchgeführt.

Die Reaktionsstufe e) wird zweckmässig bei Raumtemperatur in DMSO durchgeführt. Das entstandene 2-(1-Cyanäthyl)-6-chlorcarbazol kann in an sich bekannter Weise wie Filtrierung und Umkristallisation isoliert. Es ist jedoch nicht nötig, diese Verbindung vor ihrer Umwandlung in die 6-Chlor-α-methylcarbazol-2-essigsäure zu isolieren, vorausgesetzt, dass das Lösungsmittel abgezogen und durch ein Lösungsmittel, das für die Hydrolyse geeignet ist, d.h. ein polares Lösungsmittel, wie Wasser, Aethylenglykol,

Wasser und Aethylenglykol oder Wasser und Methanol ersetzt wird.

Die Hydrolysestufe h) wird entweder durch eine Säure, z.B. Schwefel- oder Salzsäure, oder eine Base, wie wässrigem Natriumhydroxid, in einem Alkohol, wie Methanol oder Aethylenglykol, katalysiert. Die Temperatur ist nicht kritisch und die Reaktion verläuft gut bei einer Temperatur zwischen etwa 50 und 80°C.

Nach der Hydrolyse kann man die 6-Chlor-α-methylcarbazol-2-essigsäure in an sich bekannter Weise, z.B. durch Entfernung des Lösungsmittels, durch Vakuumdestillation, Verdünnen mit Wasser und im Fall einer basischen Hydrolyse durch Versäuerung, Filtrierung, Waschen und Trocknen isolieren. Das Produkt kann durch Ausfällung aus einem organischen Lösungsmittel, vorzugsweise Aceton, in Form eines Salzes mit einer organischen Base, wie einem Trialkylamin, z.B. Trimethyl-, Tripropyl-, Tributyl-, Triisobutyl- oder vorzugsweise Triäthylamin, gefolgt durch Waschen, Trocknen und Wiederumwandlung des Produktes in die Säure, z.B. mittels Salzsäure, gereinigt werden. Das Produkt kann auch als Salz oder Säure aus einem organischen Lösungsmittel, wie Methanol/Aethylacetat im Fall eines Salzes, oder Aethylacetat/Toluol im Fall einer Säure, umkristallisiert werden.

Die Reaktionsstufe f) wird zweckmässig in Chloroform in Gegenwart einer katalytischen Menge einer Lewis-Säure, z.B. eines Zinkhalogenids, d.h. Zinkfluorid, -chlorid, -bromid oder vorzugsweise -jodid, zweckmässig unter Rückfluss und unter einer inerten Atmosphäre, z.B. unter Stickstoff oder Argon, durchgeführt.

Das entstandene 6-Chlor-α-methyl-α-tri(geradkettiges $C_{1-4}$-Alkyl)-silyloxy-9H-carbazol-2-acetonitril der Formel IV, z.B. das 6-Chlor-α-methyl-α-trimethylsilyloxy-9H--carbazol -2-acetonitril ist instabil und hat die Tendenz

sich in das Ausgangsketon umzuwandeln. Daher ist es zweckmässig, das Nitril in situ in das 2-(1-Cyanäthyl)-6-chlorcarbazol und dieses in situ in die 6-Chlor-α-methylcarbazol-2-essigsäure umzusetzen. Dies kann mittels Zinnchlorid in einem Gemisch von Salzsäure und Essigsäure bewerkstelligt werden.

Alternativ kann man das 6-Chlor-α-methyl-α-tri($C_{1-4}$-alkyl)-silyloxy -9H-carbazol-2-acetonitril in das 2-(1-Cyanäthyl)-6-chlorcarbazol unter Vermeidung von hydrolytischen Bedingungen während der Reduktion mit Zinnchlorid überführen, z.B. indem man eine Lösung von Natriumbicarbonat dem Reaktionsgemisch zusetzt und die organische Schicht vor ihrer Aufarbeitung isoliert. Die entstandene Verbindung kann man wie oben beschrieben, in die 6-Chlor-α-methylcarbazol-2-essigsäure umwandeln.

Die Erfindung betrifft die Verbindungen der Formeln II, III und IV, d.h. der Formel

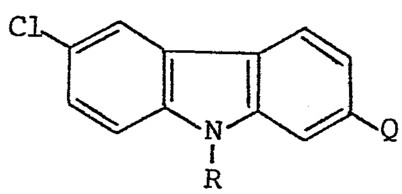

V

worin R Wasserstoff, Acetyl oder Propionyl und Q Acetyl ist, oder R Wasserstoff und Q $C(H,CH_3)OC(O)R^2$ oder $C(CN,CH_3)OSi(R^3,R^4,R^5)$ ist, wobei $R^2$ Wasserstoff, $C_{1-10}$-Alkyl, $C_{2-10}$-Alkenyl, $C_{4-7}$-Cycloalkyl, Aryl oder eine 5- oder 6-gliedrige heterocyclische Gruppe ist, und $R^3$, $R^4$ und $R^5$ geradkettige $C_{1-4}$-Alkylgruppen sind.

## Beispiel 1

100 g 2-Acetyl-6-chlorcarbazol, 10 g pulverförmiges Natriumborhydrid und 1000 ml Aethanol werden unter Rühren

erhitzt. Innerhalb von einer Stunde wurden 650 ml Aethanol abdestilliert. Danach wurden 2000 ml warmes Wasser dem Gemisch zugesetzt und das Rühren wurde 10 Minuten fortgesetzt. Nach Abkühlen wurde das Produkt durch Filtrierung, Waschen mit Wasser und Trocknen isoliert. Das entstandene 2-(1-Hydroxyäthyl)-6-chlorcarbazol (Ausbeute: 100%) war ein farbloser kristalliner Feststoff, Smp. 190-198°C.

Beispiel 2

101,3 g des Produktes von Beispiel 1, 1000 ml Aethylacetat, 100 ml Acetanhydrid und 0,50 g 4-Dimethylaminopyridin wurden im Dampfbad unter aussetzendem Rühren bis zur Bildung einer klaren Lösung erhitzt. Das Erhitzen wurde unterbrochen und die Lösung wurde drei Stunden stehen gelassen. Das Lösungsmittel wurde unter vermindertem Druck entfernt. Man erhielt das rohe Acetat und 936 ml von wiedergewonnenem Aethylacetat enthaltend Essigsäure und Acetanhydrid als Verunreinigungen. Das Produkt wurde mit 500 ml Wasser verrieben, dann auf einem Filter gewaschen und getrocknet. Man erhielt 116,35 g (98,5%) 2-(1-Acetoxyäthyl)-6-chlorcarbazol als farbloser, kristalliner Feststoff, Smp. 140-145°C.

Beispiel 3

Die 116,35 g des Produktes von Beispiel 2, 116,35 g trockenes Natriumcyanid und 750 ml trockenes DMSO wurden gerührt und unter Argon im Oelbad erhitzt. Bei einer Temperatur von 97°C und einem Druck von 2,5 mmHg wurden etwa 75 ml DMSO abdestilliert. Argon wurde in das Reaktionsgefäss eingeleitet. Die Badtemperatur wurde auf 130-132°C erhoben und die Reaktion wurde unter Rühren 9 1/2 Stunden fortgesetzt. Durch Vakuumdestillation wurden 710 ml DMSO erhalten. Das Oelbad wurde entfernt und die Reaktion wurde durch Zusatz von 500 g Eis und 500 ml kaltem Wasser unterbrochen. Das resultierende Gemisch wurde über Nacht gerührt. Das Produkt wurde filtriert und mit Wasser gewaschen, dann drei Stunden getrocknet. Man erhielt 121 g 2-(1-Cyanäthyl)-6--chlorcarbazol als feuchter leicht gelber Feststoff, Smp. 117-130°C.

## Beispiel 4

Einer gerührten Suspension von 740 ml wasserfreiem DMF und 6,36 g Lithiumhydrid wurden unter Argon vorsichtig 68 g Acetoncyanhydrin zugesetzt. Das Gemisch wurde zwei Stunden bei 50°C gerührt, mit 115,08 g 2-(1-Acetoxyäthyl)-6-chlorcarbazol behandelt und fünf Stunden unter Rückfluss gekocht. Das Lösungsmittel wurde dann unter Vakuum bei 90°C entfernt. Ein Liter warmes Wasser wurde dem Rückstand zugesetzt und das resultierende Produkt wurde gerührt. Das Produkt wurde durch Filtrieren, Waschen mit zwei Liter Wasser und Trocknen isoliert. Man erhielt 111,8 g 2-(1-Cyanäthyl)-6-chlorcarbazol als braungefärbter Feststoff von 88% Reinheit.

## Beispiel 5

Die 121 g des Produktes von Beispiel 3, 100 g Natriumhydroxid und 1000 ml Aethylenglykol wurden erhitzt und unter Argon 2 1/2 Stunden im Oelbad bei 180-185°C gerührt. Dann wurden 530 ml Aethylenglykol abdestilliert. Dem zurückbleibenden Gemisch wurden 500 g Eis und 500 ml Wasser zugesetzt. Die resultierende Lösung wurde mit 300 ml konzentrierter Salzsäure angesäuert. Nach Rühren wurde das Produkt, 6-Chlor-α-methylcarbazol-2-essigsäure, durch Filtrierung und Waschen mit 500 ml Wasser isoliert. Nach Trocknen wurden 103,3 g eines grauen Pulvers enthaltend 87 Gewichtsprozent dieser Säure (Feuchtigkeit und anorganische Salze nicht eingeschlossen) erhalten.

## Beispiel 6

Ein Gemisch von 5 ml konzentrierter Salzsäure, 10 ml Eisessig, 5 ml Wasser und 1 g 2-(1-Cyanäthyl)-6-chlorcarbazol wurden über Nacht unter Rückfluss erhitzt. Die resultierende Lösung wurde auf Raumtemperatur abkühlen gelassen und 24 Stunden gerührt. Nach Filtrierung, Waschen mit Wasser und Vakuumtrocknen erhielt man 0,88 g 6-Chlor-α-methylcarbazol-2-essigsäure (95%) in 82% Ausbeute.

## Beispiel 7

Das Produkt von Beispiel 5 wurde in 750 ml Aceton gelöst und die Lösung wurde durch Zusatz von 20 g wasserfreiem Natriumsulfat getrocknet. Die Lösung wurde filtriert und der Filterkuchen wurde mit 50 ml Aceton gewaschen. 75 ml Triäthylamin wurden dann zum Filtrat zugesetzt. Bei Beginn der Kristallisation wurde das Gemisch über Nacht im Kalten aufbewahrt. Das Salz wurde filtriert, mit Aceton gewaschen und mehrere Stunden getrocknet. Man erhielt 118,73 g (77% Ausbeute) des Triäthylaminsalzes der 6-Chlor-α-methylcarbazol-2-essigsäure als farblose Kristalle. Das Salz wurde in 400 ml Methanol gelöst und die Lösung wurde mit 110 ml 3N Salzsäure angesäuert. Die resultierende Lösung wurde mit kaltem Wasser zu 1800 ml verdünnt, mit Wasser gewaschen und getrocknet. Man erhielt 89,2 g farblosen Feststoff, enthaltend 99,0% des erwünschten Produktes. Dieses Produkt wurde umkristallisiert, durch Auflösung in 700 ml Aether und 300 ml Toluol, Entfärbung mit 10 g Holzkohle und Einengen auf etwa 250 ml durch Sieden. Nach Aufbewahren bei niedriger Temperatur wurden die Kristalle ausgeschieden und mit 200 ml Toluol gewaschen. Das Produkt wurde getrocknet und man erhielt 77,4 g farblose kristalline 6-Chlor-α-methylcarbazol-2-essigsäure verunreinigt durch eine kleine Menge Triäthylaminhydrochlorid. Zu dessen Entfernung wurde das Produkt in 225 ml heissem Methanol aufgelöst und durch Zusatz von 1400 ml Wasser ausgefällt. Das Produkt wurde ausgeschieden, mit Wasser gewaschen und getrocknet. Man erhielt 75,9 g 6-Chlor-α-methylcarbazol-2-essigsäure als farbloses Pulver, Smp. 185-188°C, Reinheit 99,4%.

## Beispiel 8

5,0 g rohe 6-Chlor-α-methylcarbazol-2-essigsäure, 25 ml Aceton und 5 ml Tributylamin wurden gemischt und 5 Minuten gerührt und dann bei Raumtemperatur stehen gelassen. Das Gemisch wurde auf 0°C abgekühlt. Das resultierende Salz wurde filtriert, mit Aceton gewaschen und getrocknet. Man erhielt 7,38 g des Tributylaminsalzes der 6-Chlor-α-methylcarbazol-2-essigsäure. Dieses Salz wurde durch Behandlung mit Methanol und Salzsäure und Umkristallisation nach dem Verfahren von Beispiel 7 in die Säure umgewandelt.

## Beispiel 9

157,0 g 2-(1-Acetoxyäthyl)-6-chlorcarbazol, 78,5 g trockenes Natriumcyanid und 790 ml trockenes DMSO wurden unter Rühren bei 100°C unter einem Vakuum von 2 mmHg erhitzt. Nachdem 80 ml DMSO abdestilliert und gesammelt wurden, wurde das Gemisch 10 Stunden unter Rühren unter einer Atmosphäre Argon bei 130°C gehalten. Das restliche DMSO wurde durch Destillation unter Vakuum gesammelt. Der Rückstand wurde abgekühlt. 120 g Natriumhydroxidkörner, 1000 ml Methanol und 200 ml Wasser wurden zugesetzt. Das Gemisch wurde auf Rückflusstemperatur (115°C) erhitzt. Es wurde weitere 24 Stunden unter Rühren in einer Argonatmosphäre unter Rückfluss erhitzt. Dann wurden 930 ml Methanol durch Destillation gesammelt. Der Rückstand wurde auf Raumtemperatur abkühlen gelassen und mit 500 ml Wasser verdünnt. Das Gemisch wurde durch Zusatz von 380 ml konzentrierter Salzsäure angesäuert. 500 ml Aethylacetat wurden dem resultierenden Schlamm zugesetzt und das Rühren wurde fortgesetzt bis eine Zweiphasenlösung entstand. Die Badtemperatur wurde

auf 35°C erhöht. 440 ml Aethylacetat wurden durch Vakuumdestillation unter Rühren gesammelt. Das Produkt 6-Chlor-α-methylcarbazol-2-essigsäure wurde in Form von Granulaten abgetrennt. Nach einer Stunde Rühren bei Raumtemperatur wurde das Produkt durch Filtrierung gesammelt, mit Wasser gewaschen und getrocknet. Es wurden 178,8 g Produkt in Form von leicht grünen Granulaten erhalten.

Beispiel 10

Ein Gemisch von 10,0 g 2-(1-Hydroxyäthyl)-6-chlorcarbazol, 50 ml Pyridin, 8,25 g Benzoylchlorid und 30 mg 4-Dimethylaminopyridin wurden zwei Stunden gerührt. Das Lösungsmittel wurde unter vermindertem Druck abgedampft. Der Rückstand wurde in 80 ml kaltem Wasser zerrieben und das Produkt wurde filtriert. Nach Waschen mit Wasser wurde der Filterkuchen über Nacht getrocknet. Man erhielt 15,75 g eines weisslichen Pulvers, das leicht mit Pyridin befeuchtet war. Umkristallisation aus Aether/Hexan ergab 12,55 g (88%) 2-(1-Benzoyloxyäthyl)-6-chlorcarbazol als farbloser kristalliner Feststoff.

Beispiel 11

Ein Gemisch von 10,0 g 2-(1-Benzoyloxyäthyl)-6-chlorcarbazol, 10,0 g trockenem Natriumcyanid und 75 ml trockenem DMSO wurde im Oelbad unter Rühren erhitzt. Der Druck wurde auf 5 mmHg reduziert. Bei 90°C wurden 20 ml DMSO abdestilliert und gesammelt. Die Badtemperatur wurde unter Argon (1 Atmosphäre) auf 128-130°C erhöht und das Gemisch wurde zwei Stunden gerührt. Das Oelbad wurde auf 100°C abgekühlt und das Vakuum wurde eingestellt bis ein Druck von 1 mmHg erreicht wurde. DMSO wurde abdestilliert. Der Behälter wurde auf Raumtemperatur abgekühlt. 100 ml heisses Wasser wurden dem Rückstand zugesetzt und das Gemisch wurde mehrere Stunden gerührt. Das Produkt wurde durch Filtrierung gesammelt, mit heissem Wasser gewaschen und getrocknet. Man erhielt

7,0 g 2-(1-Cyanäthyl)-6-chlorcarbazol als gelbes Pulver.

Beispiel 12

Ein Gemisch von 5,0 g 2-(1-Hydroxyäthyl)-6-chlorcarbazol, 50 ml Aethylacetat, 6 ml Essigsäure-Ameisensäureanhydrid und 50 mg 4-Dimethylaminopyridin wurde im Dampfbad unter Wirbel erhitzt. Die Lösung wurde dann auf Raumtemperatur abgekühlt. Abdampfen des Lösungsmittels ergab ein fester Rückstand und rohes Formiat. Dieses wurde in Wasser verrieben und filtriert. Der Filterkuchen wurde mit Wasser gewaschen und getrocknet. In einer Ausbeute von 98,5% wurde das 2-(1-Formyloxyäthyl)-6-chlorcarbazol als beiges Pulver erhalten.

Beispiel 13

5,0 g 2-(1-Formyloxyäthyl)-6-chlorcarbazol, 5,0 g Natriumcyanid und 40 ml DMSO wurden in zu Beispiel 3 analoger Weise behandelt. Man erhielt 80% 2-(1-Cyanäthyl)-6-chlorcarbazol.

Beispiel 14

Einer gerührten Lösung von 5,2 g 2-Acetyl-6-chlorcarbazol in 35 ml Chloroform, wurden unter Argon 5,08 g Trimethylsilylcyanid und 150 mg Zinkjodid zugesetzt. Das Gemisch wurde unter Rückfluss gekocht, mit zusätzlichen 2,5 g Trimethylsilylcyanid und 150 mg Zinkjodid behandelt. Das Kochen wurde 18 Stunden fortgesetzt. Das Gemisch wurde auf Raumtemperatur abgekühlt, durch Silicagel durchgelassen und eingedampft. Man erhielt 6,1 g (83% Ausbeute) 6-Chlor-$\alpha$-methyl-$\alpha$-trimethylsilyloxy-9H-carbazol -2-acetonitril. Eine Portion davon wurde durch Chromatographie auf Silicagel mit 4% Methylenchlorid in Aceton gereinigt. Man erhielt ein Feststoff, das aus Methylenchlorid-Hexan

kristallisiert wurde, Smp. 151-154°C.

Beispiel 15

171 mg 6-Chlor-α-methyl-α-trimethylsilyloxy-9H-carbazol -2-acetonitril und 5 ml Essigsäure, wurden bei Raumtemperatur unter Stickstoff gerührt. Der resultierenden Lösung wurden 450 mg Zinn-II-chloriddihydrat gefolgt von 1 ml Salzsäure zugesetzt. Das Gemisch wurde bei Raumtemperatur gerührt, dann in 150 ml gesättigtem Natriumbicarbonat geschüttet und mit 100 ml Aethylacetat extrahiert. Die organische Schicht wurde mit einer Kochsalzlösung gewaschen, getrocknet, filtriert und zur Trockene eingeengt. Das resultierende Produkt war ein Gemisch einer geringen Menge 6-Chlor-α-methyl-α-hydroxy-9H-carbazol-2-acetonitril und einer grösseren Menge 2-(1-Cyanäthyl)-6-chlorcarbazol.

Beispiel 16

Ein Gemisch von 1,71 g unreinem 6-Chlor-α-methyl--α-trimethylsilyloxy-9H-carbazol -2-acetonitril erhalten nach Beispiel 14, 2,25 g Zinn-II-chloriddihydrat und 10 ml Essigsäure wurde 10 Minuten unter Stickstoff gerührt, mit 20 ml konzentrierter Salzsäure behandelt, 18 Stunden bei 15°C und dann 2 1/2 Stunden bei 100°C gerührt. Es wurde im Vakuum eingeengt, mit 40 ml Wasser verdünnt und mit 100 ml Aethylacetat extrahiert. Der Extrakt wurde abgedampft und der Rückstand wurde mit 40 ml 3N Natriumhydroxid basisch gestellt. Nach Extraktion mit 80 ml Methylenchlorid wurde die wässrige Schicht mit konzentrierter Salzsäure angesäuert und mit 100 ml Aethylacetat extrahiert. Der Extrakt wurde mit 50 ml gesättigter Salzlösung gewaschen und mit wasserfreiem Magnesiumsulfat und 1 g Holzkohle gerührt. Das Gemisch wurde filtriert und das Filtrat wurde eingedampft. Man erhielt 1,35 g 6-Chlor-α-methylcarbazol-2-essigsäure als leicht grauer Feststoff.

## Beispiel 17

31,0 g Kaliumbutoxid wurden unter Rühren einer Lösung von 19,52 g Tosylmethylisocyanid in 150 ml DMSO unter Abkühlen im Eisbad zugesetzt. Das Gemisch wurde 5 Minuten gerührt, dann wurden 2,5 ml Methanol zugesetzt. Nach Rühren während 5 weiteren Minuten wurden 12,15 g 2-Acetyl-6-chlorcarbazol zugesetzt und die resultierende Lösung wurde bei Raumtemperatur gerührt. 150 ml Wasser, 28 ml konzentrierte Salzsäure und 100 ml Methylenchlorid wurden zugesetzt und das resultierende 2-Phasensystem wurde 5 Minuten gerührt. Die Methylenchloridschicht wurde abgetrennt und mit gesättigter Natriumbicarbonatlösung und mit Wasser gewaschen. Das Methylenchlorid wurde unter vermindertem Druck abgedampt. Der resultierende Feststoff wurde mit 200 ml Toluol verrieben und das Toluol wurde unter vermindertem Druck abgedampft. Der Rückstand wurde in 121 ml Toluol unter Rückfluss gelöst. 121 ml Hexan wurden unter Rückfluss zugesetzt und das Gemisch wurde filtriert. Die Toluol/Hexanlösung wurde unter Rühren 1/2 Stunde auf -10°C abgekühlt. Filtrierung des resultierenden Feststoffs ergab 6,6 g 2-(1-Cyanoäthyl)-6-chlorcarbazol in 53% Ausbeute.

## Beispiel 18

263 g 2,9-Diacetylcarbazol wurden mit 700 ml DMF behandelt. 100 g Trichlorisocyansäure wurden in kleinen Mengen unter ständigem Rühren zugesetzt. Die Temperatur wurde unterhalb von 40°C gehalten. Die resultierende Lösung wurde eine Stunde gerührt und dann wurde 2 Minuten gasförmiges Chlorwasserstoff in den Raum über die Lösung zugeleitet. Die Lösung wurde im Wasserbad abgekühlt, bei Raumtemperatur gerührt und dann 3 Stunden abgekühlt. Die Abtrennung des Produktes durch Nutschen wurde in einem kalten Raum durchgeführt. Das Filtrat wurde zum Spühlen aller Feststoffe verwendet und der Filterkuchen wurde durch Pressen vom DMF befreit. Das Produkt wurde in 1000 ml heisser Essigsäure aufgenommen und filtriert. Der Feststoff wurde mit 50 ml heisser Essigsäure gewaschen. Das Filtrat und die Waschwässer wurden bei Raumtemperatur belassen. Das auskristallisierte Produkt wurde filtriert, mit 150 ml Essigsäure gewaschen und getrocknet. Man erhielt 159,38 g 6-Chlor-2,9-diacetylcarbazol als blassgelbe Kristalle, Smp. 154-158°C.

## Beispiel 19

Ein Gemisch von 159,38 g des umkristallisierten 6-Chlor-2,9-diacetylcarbazol, 1600 ml Aethanol und 100 ml einer 4,4M Lösung von Natriumborhydrid in 14N Natriumhydroxid wurde unter Rückfluss erhitzt. 1320 ml des verwendeten Aethanols wurden durch Destillation rückgewonnen. Der Rückstand wurde in 2500 ml warmem Wasser aufgeschlämmt und über Nacht stehen gelassen. Das Produkt wurde filtriert und mit Wasser gewaschen, bis die Waschwässer neutral waren. Das Produkt wurde trockengepresst und bei 70°C getrocknet. Man erhielt 134,3 g (98%) 2-(1-Hydroxyäthyl)-6-chlorcarbazol als farbloses Pulver, Smp. 194-198°C.

## Beispiel 20

Ein Gemisch von einem Liter wasserfreiem Triäthylphosphat und 52,25 g 2-Acetylcarbazol wurde unter Stickstoff unter Rühren erwärmt. Die resultierende Lösung wurde auf Raumtemperatur abgekühlt, mit 1,5 ml 48%-iger Fluorborsäure behandelt und auf 5°C abgekühlt. 21,6 g Trichlorisocyansäure in 250 ml wasserfreiem Triäthylphosphat wurden dann tropfenweise zugesetzt. Das Gemisch wurde eine Stunde bei 5°C gerührt und drei Stunden bei Raumtemperatur. Es wurde dann im Vakuum im Dampfbad eingeengt. Dem Rückstand wurde ein Liter 1N Natriumhydroxid unter Rühren zugesetzt. Das Produkt wurde filtriert und mit Wasser gewaschen und getrocknet. Man erhielt 61 g 2-Acetyl-6-chlorcarbazol. Dieses wurde bei 130°C in 110 ml DMF aufgelöst und mit Holzkohle behandelt. Das Gemisch wurde filtriert und der Filterkuchen wurde mit 30 ml

DMF gewaschen. Das Filtrat und die Waschwässer wurden unter Rühren erhitzt. Die erhaltene Lösung wurde 280 ml heissem Acetonitril zugesetzt. Das Gemisch wurde unter Rühren auf Raumtemperatur, dann auf 10°C abgekühlt. Das Produkt wurde filtriert, mit Acetonitril gewaschen und im Vakuum bei 90°C getrocknet. Man erhielt 41,7 g 2-Acetyl-6-chlorcarbazol als gelbe Kristalle, Smp. 250-251°C.

## Beispiel 21

Ein Gemisch von 52,25 g 2-Acetylcarbazol und einem Liter wasserfreiem Triäthylphosphat wurde gerührt und unter Stickstoff erwärmt, dann auf 5°C abgekühlt und mit 5 ml Bortrifluoridätherat behandelt. Das Gemisch wurde 15 Minuten gerührt und tropfenweise mit einer Lösung von 22 g Trichlorisocyansäure in 250 ml wasserfreiem Triäthylphosphat behandelt. Das Gemisch wurde eine Stunde bei 0-5°C und vier Stunden bei Raumtemperatur gerührt. Es wurde dann im Vakuum im Dampfbad eingengt. Dem Rückstand wurde ein Liter 1N Natriumhydroxid unter Rühren zugesetzt. Das Produkt wurde filtriert und mit Wasser gewaschen und getrocknet. Man erhielt 61 g 2-Acetyl-6-chlorcarbazol die in 110 ml DMF bei 130°C aufgelöst und mit Holzkohle behandelt wurden. Das Gemisch wurde filtriert und der Filterkuchen wurde mit 30 ml DMF gewaschen. Das Filtrat und die Waschwässer wurden unter Rühren erhitzt. Der erhaltenen Lösung wurden 280 ml heisses Acetonitril zugesetzt. Das Gemisch wurde unter Rühren auf Raumtemperatur und dann auf 10°C abgekühlt. Das Produkt wurde filtriert, mit Acetonitril gewaschen und im Vakuum bei 90°C getrocknet. Man erhielt 41,7 g 2-Acetyl-6-chlorcarbazol als gelbe Kristalle, Smp. 250-251°C.

## Patentansprüche

1. Verfahren zur Herstellung der 6-Chlor-α-methylcarbazol-2-essigsäure, dadurch gekennzeichnet, dass man

a) eine Verbindung der Formel

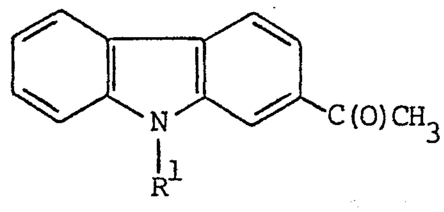

I

worin $R^1$ Wasserstoff, Acetyl oder Propionyl ist, chloriert und entweder

b) die entstandene Verbindung der Formel

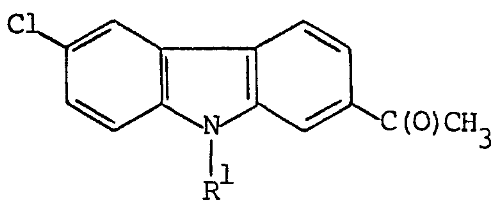

II

worin $R^1$ die obige Bedeutung hat mit einem Reduktionsmittel umsetzt

c) die entstandene Verbindung, 2-(1-Hydroxyäthyl)-6-chlorcarbazol, acyliert und

d) die entstandene Verbindung der Formel

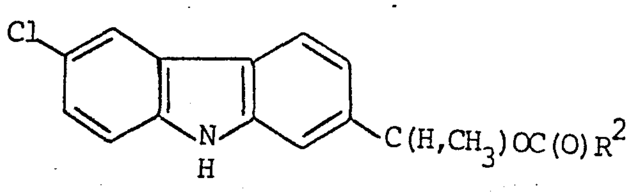

III

worin $R^2$ Wasserstoff, $C_{1-10}$-Alkyl, $C_{2-10}$-Alkenyl, $C_{4-7}$-Cycloalkyl, Aryl oder ein 5- oder 6-gliedrige heterocyclische Gruppe ist,

mit einem Verschiebungsmittel umsetzt, oder

e) die Verbindung der Formel II, worin $R^1$ Wasserstoff ist, 2-Acetyl-6-chlorcarbazol, mit Tosylmethylisocyanid in Gegenwart von Kalium-t-butoxid umsetzt oder

f) 2-Acetyl-6-chlorcarbazol mit einem tri-(geradkettigen $C_{1-4}$-Alkyl)-silylcyanid umsetzt und

g) die entstandene Verbindung der Formel

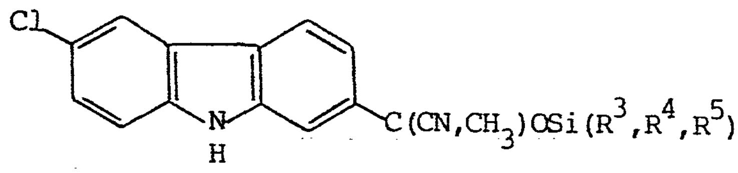

IV

worin $R^3$, $R^4$ und $R^5$ geradkettige $C_{1-4}$-Alkylgruppen sind,

mit Zinnchlorid umsetzt und

h) das Produkt der Verfahrensstufe d), e) oder g), 2-(1-Cyanoäthyl)-6-chlorcarbazol, hydrolysiert.

2. Verfahren zur Herstellung einer Verbindung der Formel

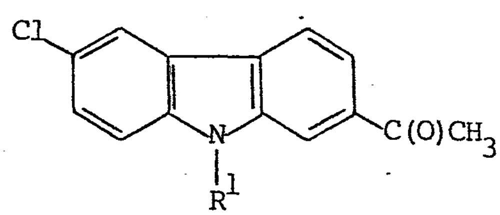

II

worin $R^1$ Wasserstoff, Acetyl oder Propionyl ist,

dadurch gekennzeichnet, dass man entweder

a) 2,9-Diacetyl- oder 2-Acetyl-9-Propionyl-carbazol mit Trichlorisocyansäure, Sulfonylchlorid oder Natriumhypochlorit in einem polaren Lösungsmittel, nämlich Dimethylformamid, 1,2-Dichloräthan oder Triäthylphosphat, umsetzt oder

b) 2-Acetylcarbazol mit 1-Chlorbenzotriazol oder Trichlorisocyansäure in einem polaren Lösungsmittel, nämlich Methylenchlorid oder 1,2-Dichloräthan, umsetzt.

3. Verfahren zur Herstellung des 2-(1-Cyanäthyl)--6-chlorcarbazols, dadurch gekennzeichnet, dass man

a) eine Verbindung der Formel

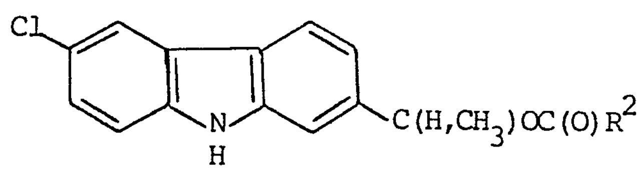

III

worin $R^2$ Wasserstoff, $C_{1-10}$-Alkyl, $C_{2-10}$-Alkenyl, $C_{4-7}$-Cycloalkyl, Aryl oder ein 5- bis 6-gliedrige heterocyclische Gruppe ist,
mit einem Alkalimetallcyanid unter praktisch wasserfreien Bedingungen in einem polaren aprotischen Lösungsmittel umsetzt oder

b) 2-Acetyl-6-chlorcarbazol mit Tosylmethylisocyanid in Gegenwart von Kalium-t-butoxid in einer inerten Atmosphäre umsetzt.